(19) Europäisches Patentamt
European Patent Office
Office européen des brevets

(11) Veröffentlichungsnummer : **0 141 319 B1**

(12) # EUROPÄISCHE PATENTSCHRIFT

(45) Veröffentlichungstag der Patentschrift : **25.05.88**

(51) Int. Cl.[4] : **C 07 D263/58**, C 07 D277/68, A 01 N 43/76, A 01 N 43/70

(21) Anmeldenummer : **84112291.4**

(22) Anmeldetag : **12.10.84**

(54) **Substituierte Phenoxyalkancarbonsäureester.**

(30) Priorität : **25.10.83 JP 198235/83**

(43) Veröffentlichungstag der Anmeldung : **15.05.85 Patentblatt 85/20**

(45) Bekanntmachung des Hinweises auf die Patenterteilung : **25.05.88 Patentblatt 88/21**

(84) Benannte Vertragsstaaten : **BE CH DE FR GB IT LI NL**

(56) Entgegenhaltungen :
**EP-A- 0 062 905**
**DE-A- 2 830 066**
**GB-A- 2 046 753**
**US-A- 4 130 413**
**Die Akte enthält technische Angaben, die nach dem Eingang der Anmeldung eingereicht wurden und die nicht in dieser Patentschrift enthalten sind.**

(73) Patentinhaber : **NIHON TOKUSHU NOYAKU SEIZO K.K.**
**No.4, 2-chome, Nihonbashi Honcho Chuo-ku**
**Tokyo 103 (JP)**

(72) Erfinder : **Shiokawa, Kozo**
**210-6, Shukugawara Tama-ku**
**Kawasaki-shi Kanagawa (JP)**
Erfinder : **Moriya, Koichi**
**39-15, Namiki-cho**
**Hachioji-shi Tokyo (JP)**
Erfinder : **Goto, Toshio**
**20-1-304, 5-chome, Seishin**
**Sagamihara-shi Kanagawa-ken (JP)**
Erfinder : **Kamochi, Atsumi**
**2-24-10, Higashi-Toyoda**
**Hino-shi Tokyo (JP)**
Erfinder : **Ito, Seishi**
**39-15, Namiki-cho**
**Hachioji-shi Tokyo (JP)**
Erfinder : **Horita, Mitsugu**
**3-19-6, Toyoda**
**Hino-shi Tokyo (JP)**

(74) Vertreter : **Schumacher, Günter, Dr. et al**
**c/o Bayer AG Konzernverwaltung RP Patentabteilung**
**D-5090 Leverkusen 1 Bayerwerk (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die vorliegende Erfindung betrifft neue substituierte Phenoxyalkancarbonsäureester, mehrere Verfahren zu ihrer Herstellung und ihre Verwendung als Herbizide.

Es ist bereits bekannt, daß bestimmte Benzoxazolyl- und Benzthiazolyl-phenoxy-propionsäure-Derivate herbizide Eigenschaften besitzen (vgl. US-PS 4 130 413 und Japanische Offenlegungsschrift 40 767-1978). So kann zum Beispiel 2-(4-[(6-Chlor-benzthiazol-2-yl)-oxy]-phenoxy)-propionsäure-ethylester der Formel A-1

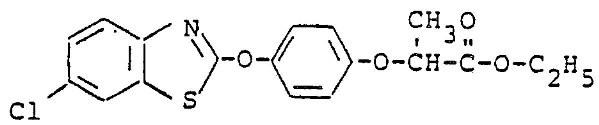
(A-1)

zur Bekämpfung von Unkräutern verwendet werden. Die Wirkung dieses Stoffes ist jedoch nicht immer völlig ausreichend.

Es wurden jetzt neue substituierte Phenoxyalkancarbonsäureester der Formel I,

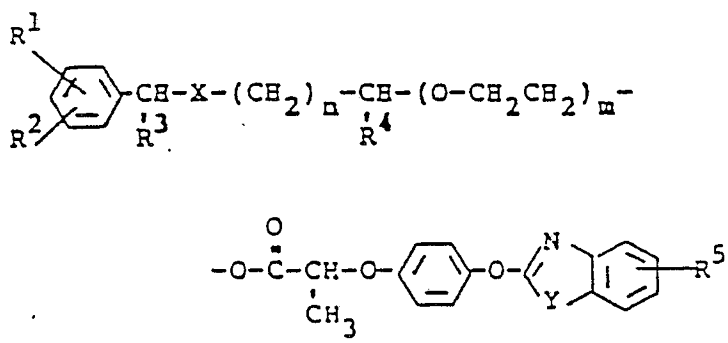
(I)

in welcher

$R^1$ und $R^2$ unabhängig voneinander für Wasserstoff, Fluor, Chlor, Brom, Jod, Alkyl mit 1 bis 6 Kohlenstoffatomen oder Nitro stehen, oder

$R^1$ und $R^2$ gemeinsam mit dem Benzolring an den sie gebunden sind, einen Naphthalin-Ring bilden,

$R^3$ und $R^4$ unabhängig voneinander für Wasserstoff oder Methyl stehen,

m für 0 oder 1 steht,

n für 1 oder 2 steht,

X für Sauerstoff, Schwefel, eine Sulfonylgruppe oder eine Gruppe der Formel

$$-\overset{|}{N}-R^6$$

steht, worin $R^6$ für Alkyl mit 1 bis 6 Kohlenstoffatomen steht,

$R^5$ für Wasserstoff, Fluor, Chlor, Brom oder Jod steht und

Y für Sauerstoff oder Schwefel steht,

gefunden.

Die substituierten Phenoxyalkancarbonsäureester der Formel I enthalten mindestens ein asymmetrisch substituiertes Kohlenstoffatom und können deshalb in verschiedenen enantiomeren Formen vorliegen. Die Erfindung betrifft sowohl die einzelnen möglichen Isomeren als auch Gemische dieser Isomeren.

Weiterhin wurde gefunden, daß man substituierte Phenoxyalkancarbonsäureester der Formel I erhält, wenn man

a) Verbindungen der Formel II,

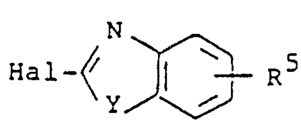
(II)

in welcher

$R^5$ und Y die oben angegebene Bedeutung haben und

Hal für Halogen steht,

mit Verbindungen der Formel III,

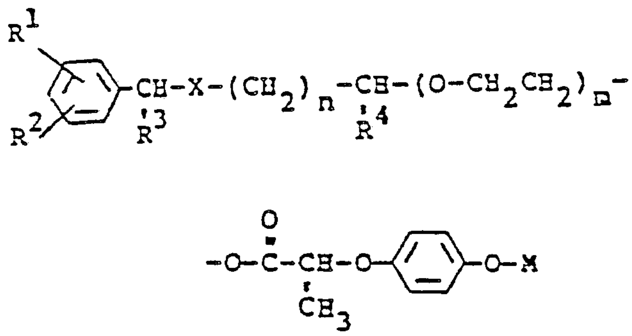

(III)

in welcher
$R^1$, $R^2$, $R^3$, $R^4$, X, m und n die oben angegebene Bedeutung haben und
M für Wasserstoff oder ein Alkalimetallatom steht,
umsetzt,
oder

b) Verbindungen der Formel IV,

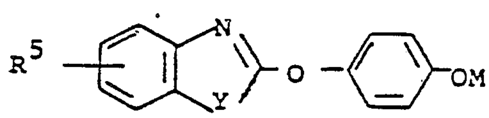

(IV)

in welcher
$R^5$ und Y die oben angegebene Bedeutung haben und
M für Wasserstoff oder ein Alkalimetallatom steht,
mit Verbindungen der Formel V,

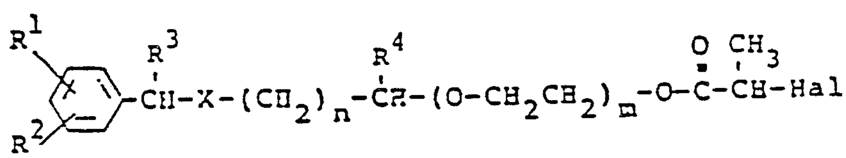

(V)

in welcher
$R^1$, $R^2$, $R^3$, $R^4$, X, m und n die oben angegebene Bedeutung haben und
Hal für Halogen steht,
umsetzt,
oder

c) Verbindungen der Formel VI,

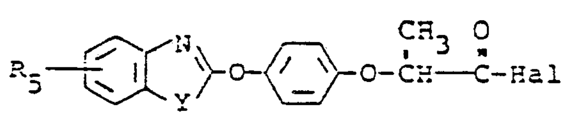

(VI)

in welcher
$R^5$ und Y die oben angegebene Bedeutung haben und
Hal für Halogen steht,
mit Verbindungen der Formel VII,

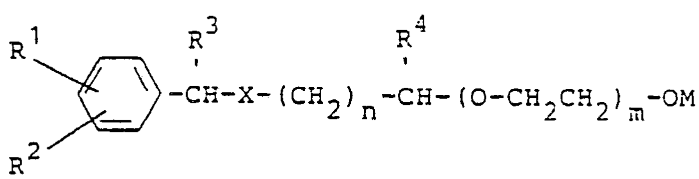

(VII)

in welcher

$R^1$, $R^2$, $R^3$, $R^4$, X, m und n die oben angegebene Bedeutung haben und
M für Wasserstoff oder ein Alkalimetallatom steht,

umsetzt

oder

d) Verbindungen der Formel Ia,

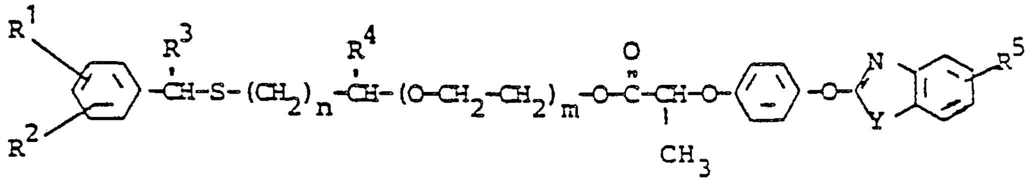

(Ia)

in welcher $R^1$, $R^2$, $R^3$, $R^4$, $R^5$, Y, m und n die oben angegebene Bedeutung haben, mit Wasserstoffperoxid umsetzt.

Schließlich wurde gefunden, daß sich die erfindungsgemäßen substituierten Phenoxyalkancarbonsäureester der Formel I durch sehr gute herbizide Eigenschaften auszeichnen. Die vorliegende Erfindung betrifft deshalb auch herbizide Mittel, die substituierte Phenoxyalkancarbonsäureester der Formel I als Wirkstoffe enthalten. Außerdem betrifft die vorliegende Erfindung ein Verfahren zur Bekämpfung von Unkräutern, welches darin besteht, daß man substituierte Phenoxyalkancarbonsäureester der Formel I auf die Pflanzen und/oder deren Lebensraum ausbringt.

Überraschenderweise besitzen die erfindungsgemäßen Stoffe der Formel I, eine ausgezeichnete herbizide Wirksamkeit. Sie sind besonders vorteilhaft einsetzbar zur selektiven Bekämpfung einer großen Anzahl von Unkräutern, wie zum Beispiel Unkräuter aus der Familie der Graminaceae, ohne eine nennenswerte Phytotoxizität gegenüber landwirtschaftlichen Nutzpflanzen zu entfalten. Es ist besonders überraschend, daß die erfindungsgemäßen Verbindungen der Formel I auch eine bessere herbizide Wirksamkeit aufweisen als der 2-{4-[(6-Chlor-benzthiazol-2-yl)-oxy]-phenoxy}-propionsäure-ethylester, welcher ein konstitutionell ähnlicher, aus dem Stand der Technik bekannter Wirkstoff gleicher Wirkungsart ist. Vor allem ist hervorzuheben, daß die erfindungsgemäßen substituierten Phenoxyalkancarbonsäureester der Formel I auch dann, wenn sie in niedrigen Aufwandmengen eingesetzt werden, eine hervorragende Selektivität besitzen und außerdem auch der Regenerierung von Unkräutern, insbesondere von perennierenden grasartigen Unkräutern, infolge ihrer ausgezeichneten Langzeitwirkung über eine lange Zeitdauer hinweg erfolgreich entgegenwirken.

Die erfindungsgemäßen Verbindungen der Formel I sind strukturell dadurch charakterisiert, daß die nachstehende Gruppe

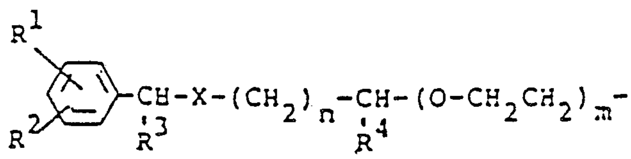

über eine Ester-Verknüpfung an Benzo-azoyloxyphenoxypropionsäure gebunden ist.

Bevorzugte erfindungsgemäße Wirkstoffe sind diejenigen substituierten Phenoxyalkancarbonsäureester der Formel I, in denen

$R^1$ und $R^2$ unabhängig voneinander für Wasserstoff, Fluor, Chlor, Brom, Methyl, Ethyl, n-Propyl, Isopropyl, n-Butyl, iso-Butyl, sek-Butyl, tert-Butyl oder Nitro stehen, oder
$R^1$ und $R^2$ gemeinsam mit dem Benzolring, an den sie gebunden sind, einen Naphthalin-Ring bilden,
$R^3$ und $R^4$ unabhängig voneinander für Wasserstoff oder Methyl stehen,
m für 0 oder 1 steht,
n für 1 oder 2 steht,
X für Sauerstoff, Schwefel, eine Sulfonylgruppe oder eine Gruppe der Formel $-\overset{|}{N}-R^6$ steht, worin $R^6$ für Methyl, Ethyl, n-Propyl, iso-Propyl, n-Butyl, iso-Butyl, sek-Butyl oder tert-Butyl steht,
$R^5$ für Wasserstoff, Fluor, Chlor oder Brom steht und
Y für Sauerstoff oder Schwefel steht.

Wie bereits oben erwähnt wurde, erhalten die erfindungsgemäßen Verbindungen mindestens ein asymmetrisch substituiertes Kohlenstoffatom in der Seitenkette und können deshalb in zwei enantiomeren Formen vorliegen. In der nachfolgenden Formel ist das asymmetrisch substituierte Kohlenatom durch ein (*) gekennzeichnet.

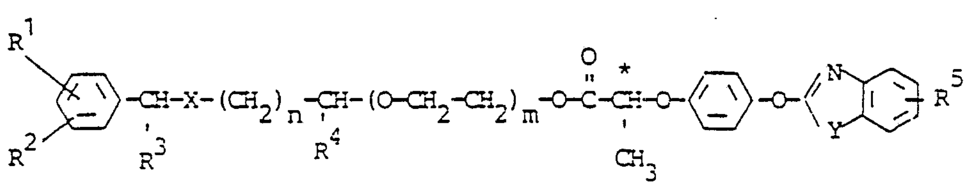

Die Erfindung betrifft sowohl die jeweiligen Racemate als auch die R- und S-Enantiomeren.

Unter R-Enantiomeren (S-Enantiomeren) sind hierbei jeweils diejenigen optisch aktiven Verbindungen zu verstehen, welche am asymmetrisch substituierten Kohlenstoffatom der Propionsäure-Einheit die R-Konfiguration (S-Konfiguration) aufweisen.

Bevorzugt sind die R-Enantiomeren derjenigen substituierten Phenoxyalkancarbonsäureester der Formel I, in denen $R^1$, $R^2$, $R^3$, $R^4$, $R^5$, X, Y, m und n die oben als bevorzugt genannten Bedeutungen haben.

Der Verlauf des erfindungsgemäßen Verfahrens (a) kann durch das folgende Reaktionsschema veranschaulicht werden :

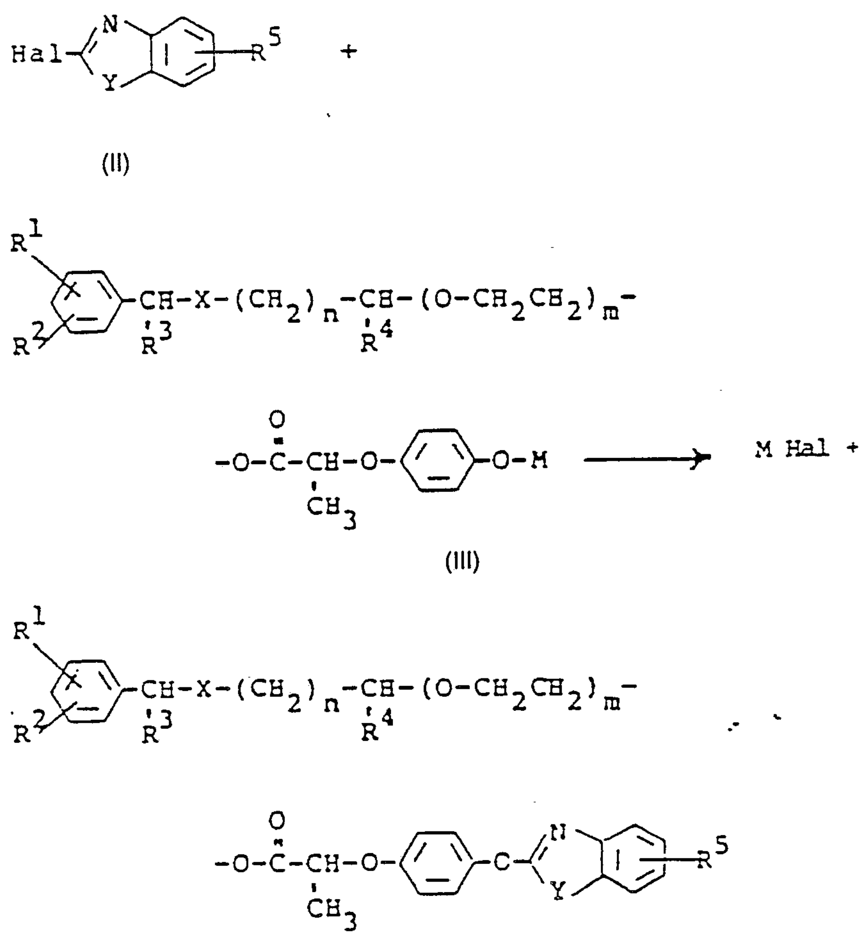

(II)

(III)

(I)

Das vorstehende Verfahren (a) wird anhand des folgenden typischen Beispiels speziell beschrieben.

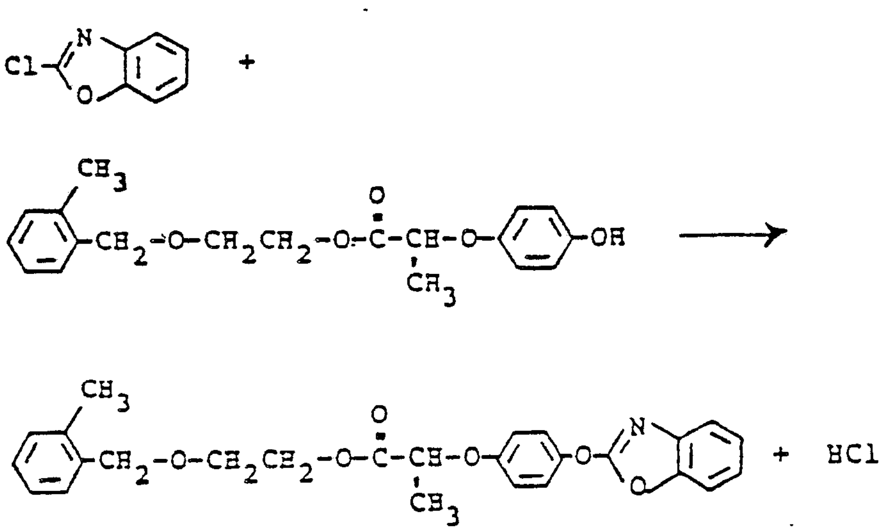

Die bei dem erfindungsgemäßen Verfahren (a) als Ausgangsstoffe benötigten Verbindungen sind durch die Formel II definiert. In dieser Formel haben $R^5$ und Y vorzugsweise diejenigen Bedeutungen, die bereits im Zusammenhang mit der Beschreibung der erfindungsgemäßen Stoffe der Formel I für diese Reste als bevorzugt genannt wurden. Hal steht vorzugsweise für Chlor oder Brom.

Als Beispiele für Verbindungen der Formel II seien genannt :
2-Chlorobenzothiazol,
2-Bromobenzothiazol,
2-Chlorobenzoxazol,
2-Bromobenzoxazol und
6-Chloro-2-bromobenzothiazol.

Die bei dem erfindungsgemäßen Verfahren (a) weiterhin als Ausgangsstoffe benötigten Verbindungen sind durch die Formel III definiert. In dieser Formel haben $R^1$, $R^2$, $R^3$, $R^4$, X, m und n vorzugsweise diejenigen Bedeutungen die bereits im Zusammenhang mit der Beschreibung der erfindungsgemäßen Stoffe der Formel I für diese Reste und Indices als bevorzugt genannt wurden. M steht vorzugsweise für Wasserstoff, Lithium, Natrium und Kalium.

Als Beispiele für Verbindungen der Formel III seien genannt :
2-(2-Chlorobenzyloxy)ethyl-2-(4-hydroxyphenoxy)-propionat,
2-(4-Chlorobenzyloxy)ethyl-2-(4-hydroxyphenoxy)-propionat,
2-(2,6-Dichlorobenzyloxy)ethyl-2-(4-hydroxyphenoxy)-propionat,
2-(2-Fluorobenzyloxy)ethyl-2-(4-hydroxyphenoxy)-propionat,
2-(2-Methylbenzyloxy)ethyl-2-(4-hydroxyphenoxy)-propionat,
2-(1-Napthylmethoxy)ethyl-2-(4-hydroxyphenoxy)-propionat,
2-(3-Nitrobenzyloxy)ethyl-2-(4-hydroxyphenoxy)-propionat,
2-(N-Benzyl-N-isopropylamino)ethyl-2-(4-hydroxyphenoxy)-propionat,
2-Benzyloxyethyl-2-(4-hydroxyphenoxy)-propionat,
2-(α-Methylbenzyloxy)ethyl-2-(4-hydroxyphenoxy)-propionat,
2-(2-Benzyloxyethoxy)ethyl-2-(4-hydroxyphenoxy)-propionat,
2-(1-Methyl-2-benzyloxy)ethyl-2-(4-hydroxyphenoxy)-propionat,
3-Benzyloxypropyl-2-(4-hydroxyphenoxy)-propionat,
2-Benzylthioethyl-2-(4-hydroxyphenoxy)-propionat,
2-(2-Chlorobenzylthio)ethyl-2-(4-hydroxyphenoxy)-propionat,
2-(2-Chlorobenzylsulfonyl)ethyl-2-(4-hydroxyphenoxy)-propionat und
2-(N-Benzyl-N-methylamino)ethyl-2-(4-hydroxyphenoxy)-propionat.

Die entsprechenden Alkalimetall-Salze wie die Lithium-, Natrium- und Kalium-Salze können ebenfalls als Beispiele genannt werden.

Die Umsetzung nach dem erfindungsgemäßen Verfahren (a) wird vorzugsweise in Gengenwart eines Lösungsmittels oder eines Verdünnungsmittels durchgeführt. Zu diesem Zweck können sämtliche inerten Lösungsmittel und Verdünnungsmittel verwendet werden.

Beispiele für solche Lösungsmittel oder Verdünnungsmittel umfassen Wasser, aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol ; Ether wie Diethylether, Methylethylether, Diisopropylether, Dibutylether, Propylenoxid, Dioxan und Tetrahydrofuran ; Ketone wie Aceton, Methylethylketon, Methylisopropylketon und Methylisobutylketon ; Nitrile wie Acetonitril, Propionitril und Acrylnitril ; Alkohole wie Methanol, Ethanol, Isopropanol, Butanol und Ethylenglycol ; Ester wie Ethylacetat und Amylacetat ; Säureamide wie Dimethylformamid und Dimethylacetamid ; Sulfone und Sulfoxide wie Dimethylsulfoxid und Sulfolan ; sowie Basen wie Pyridin.

Die obige Reaktion nach dem erfindungsgemäßen Verfahren (a) kann in Gegenwart eines säurebindenden Mittels durchgeführt werden. Beispiele für das säurebindende Mittel umfassen die Hydroxide, Carbonate, Hydrogencarbonate und Alkoholate von Alkalimetallen, sowie tertiäre Amine wie Triethylamin, Diethylanilin und Pyridin, die sämtlich allgemein verwendet werden.

Das erfindungsgemäße Verfahren (a) kann in einem weiten Temperaturbereich durchgeführt werden. Im allgemeinen kann es bei einer Temperatur zwischen etwa — 20 °C und dem Siedepunkt der Mischung, zweckmäßigerweise zwischen 0 °C und etwa 100 °C, durchgeführt werden. Die Reaktion wird zweckmäßigerweise unter Atmosphärendruck durchgeführt, jedoch ist es auch möglich, unter erhöhtem oder vermindertem Druck zu arbeiten.

Der Verlauf des erfindungsgemäßen Verfahrens (b) kann durch das folgende Reaktionsschema veranschaulicht werden :

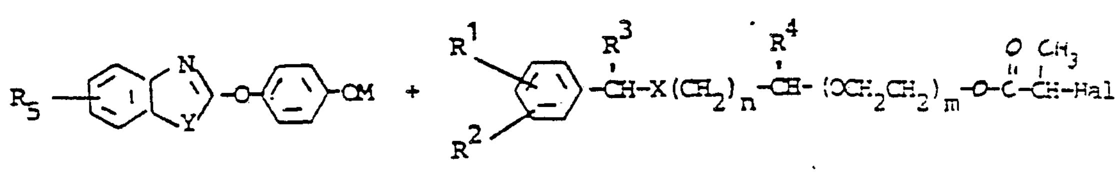

(IV) (V)

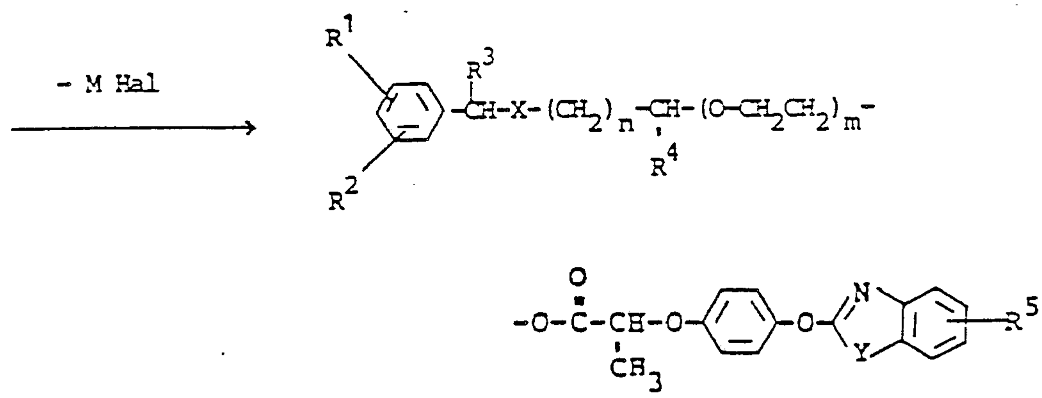


(I)

Das vorstehende Verfahren (b) wird anhand des folgenden typischen Beispiels speziell beschrieben.

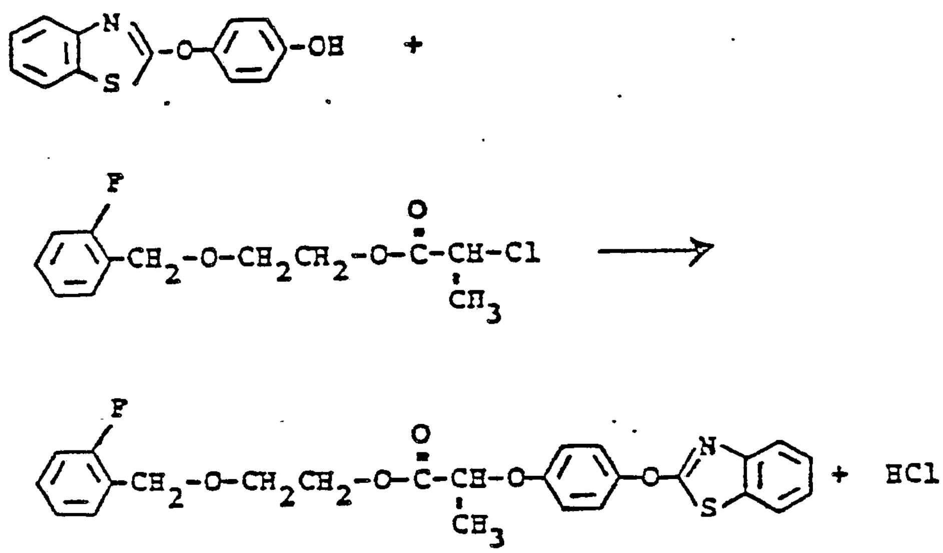


Die bei dem erfindungsgemäßen Verfahren (b) als Ausgangsstoffe benötigten Verbindungen sind durch die Formel IV definiert. In dieser Formel haben $R^5$ und Y vorzugsweise diejenigen Bedeutungen, die bereits im Zusammenhang mit der Beschreibung der erfindungsgemäßen Stoffe der Formel I für diese Reste als bevorzugt genannt wurden. M steht vorzugsweise für Wasserstoff, Lithium, Natrium oder Kalium.

Die bei dem erfindungsgemäßen Verfahren (b) weiterhin als Ausgangsstoffe benötigten Verbindungen sind durch die Formel V definiert. In dieser Formel haben $R^1$, $R^2$, $R^3$, $R^4$, X, m und n vorzugsweise diejenigen Bedeutungen, die bereits im Zusammenhang mit der Beschreibung der erfindungsgemäßen Stoffe der Formel I für diese Reste und Indices als bevorzugt genannt wurden. Hal steht vorzugsweise für Chlor oder Brom.

Bei der Durchführung des erfindungsgemäßen Verfahrens (b) können mit Ausnahme von Alkoholen vorzugsweise alle diejenigen inerten Solventien und Verdünnungsmittel verwendet werden, die bereits im Zusammenhang mit der Durchführung des erfindungsgemäßen Verfahrens (a) genannt wurden. Die Endprodukte werden dabei in hoher Ausbeute und sehr guter Reinheit erhalten. Ebenso können bei der Durchführung des erfindungsgemäßen Verfahrens (b) vorzugsweise alle diejenigen Säurebindemittel eingesetzt werden, die bereits im Zusammenhang mit der Beschreibung des erfindungsgemäßen Verfahrens (a) genannt wurden.

Die Umsetzung nach dem erfindungsgemäßen Verfahren (b) kann innerhalb eines größeren Temperaturbereiches durchgeführt werden. Im allgemeinen arbeitet man bei Temperaturen zwischen —20 °C und dem Siedepunkt des Reaktionsgemisches, vorzugsweise zwischen 0 °C und 100 °C.

Die Umsetzung nach dem erfindungsgemäßen Verfahren (b) wird vorzugsweise unter atmosphärischem Druck durchgeführt, sie kann jedoch auch unter erhöhtem oder vermindertem Druck vorgenommen werden.

Der Verlauf des erfindungsgemäßen Verfahrens (c) kann durch das folgende Reaktionsschema veranschaulicht werden.

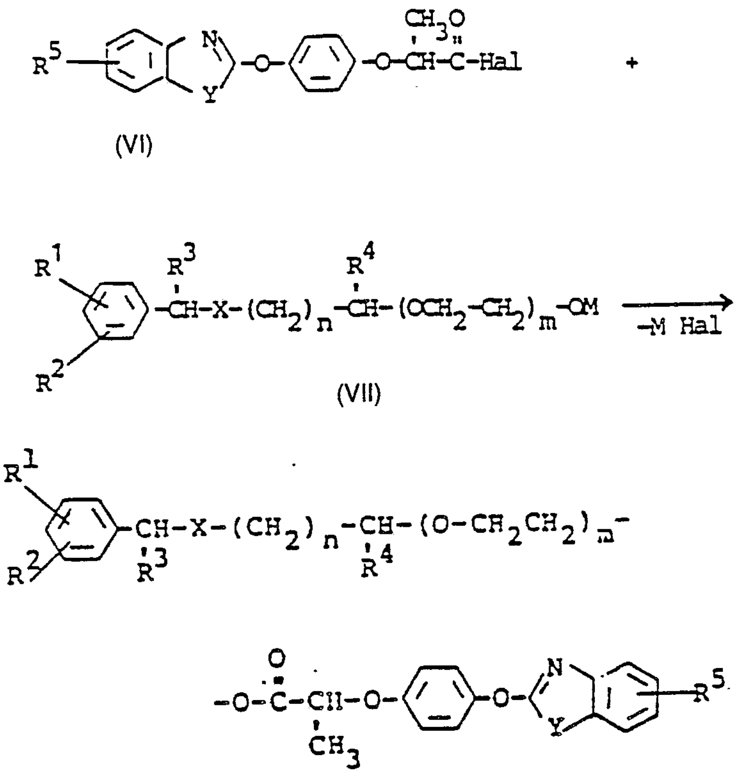


(VI)

(VII)

Das vorstehende Verfahren (c) wird anhand des folgenden typischen Beispiels speziell beschrieben.

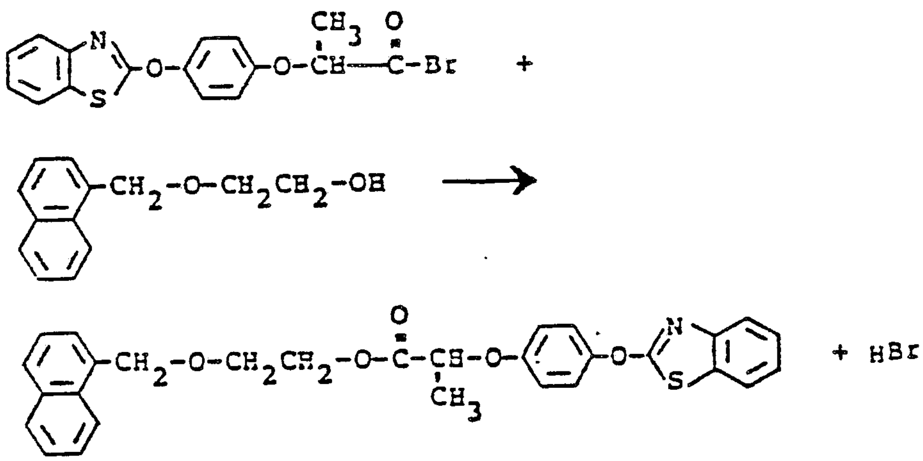

Die bei dem erfindungsgemäßen Verfahren (c) als Ausgangsstoffe benötigten Verbindungen sind durch die Formel VI definiert. In dieser Formel haben $R^5$ und Y vorzugsweise diejenigen Bedeutungen, die bereits im Zusammenhang mit der Beschreibung der erfindungsgemäßen Stoffe der Formel I für dieser Reste als bevorzugt genannt wurden. Hal steht vorzugsweise für Chlor oder Brom.

Die bei dem erfindungsgemäßen Verfahren (c) weiterhin als Ausgangsstoffe benötigten Verbindungen sind durch die Formel VII definiert. In dieser Formel haben $R^1$, $R^2$, $R^3$, $R^4$, X, m und n vorzugsweise diejenigen Bedeutungen, die bereits im Zusammenhang mit der Beschreibung der erfindungsgemäßen Stoffe der Formel I für diese Reste und Indices als bevorzugt genannt wurden. M steht vorzugsweise für Wasserstoff, Lithium, Natrium und Kalium.

Bei der Durchführung des erfindungsgemäßen Verfahrens (c) können mit Ausnahme von Alkoholen vorzugsweise alle diejenigen inerten Solventien und Verdünnungsmittel verwendet werden, die bereits im Zusammenhang mit der Durchführung des erfindungsgemäßen Verfahrens (a) genannt wurden. Die Endprodukte werden dabei in hoher Ausbeute und sehr guter Reinheit erhalten. Ebenso können bei der Durchführung des erfindungsgemäßen Verfahrens (c) vorzugsweise alle diejenigen Säurebindemittel eingesetzt werden, die bereits im Zusammenhang mit der Beschreibung des erfindungsgemäßen Verfahrens (a) genannt wurden.

Die Umsetzung nach dem erfindungsgemäßen Verfahren (c) kann innerhalb eines größeren Temperaturbereiches durchgeführt werden. Im allgemeinen arbeitet man bei Temperaturen zwischen

— 20 °C und dem Siedepunkt des Reaktionsgemisches, vorzugsweise zwischen 0 °C und 100 °C.

Die Umsetzung nach dem erfindungsgemäßen Verfahren (c) wird vorzugsweise unter atmosphärischem Druck durchgeführt ; sie kann jedoch auch unter erhöhtem oder vermindertem Druck vorgenommen werden.

Der Verlauf des erfindungsgemäßen Verfahrens (d) kann durch das folgende Reaktionsschema veranschaulicht werden.

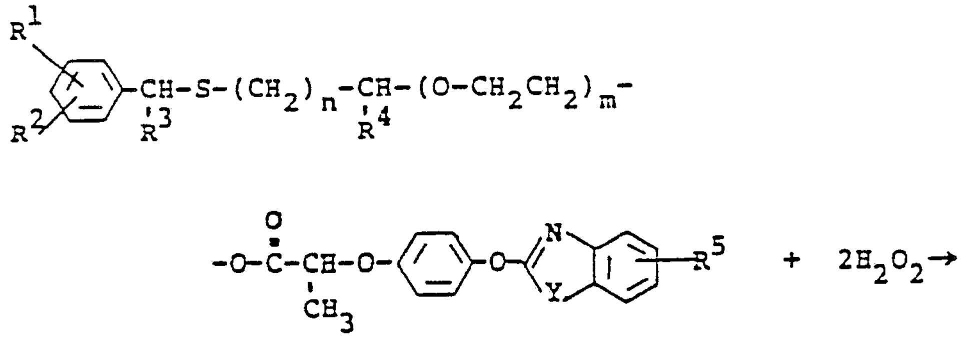

(Ia)

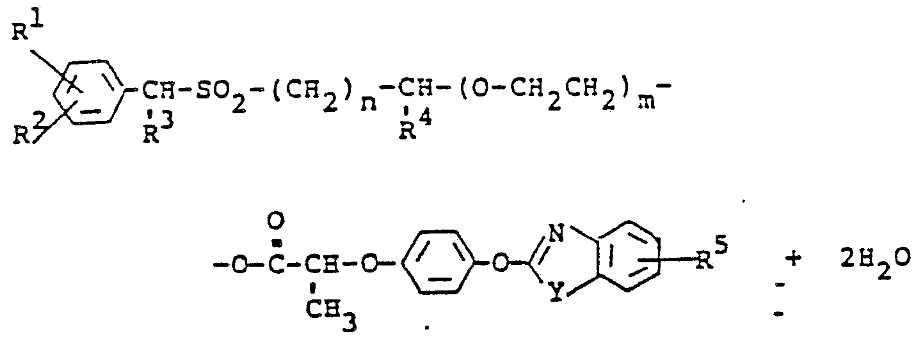

(Ib)

Das vorstehende Verfahren (d) wird anhand des folgenden typischen Beispiels speziell beschrieben.

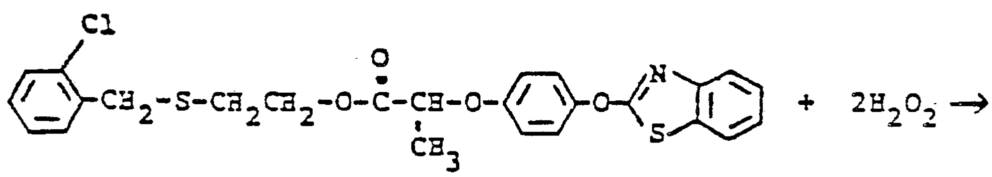

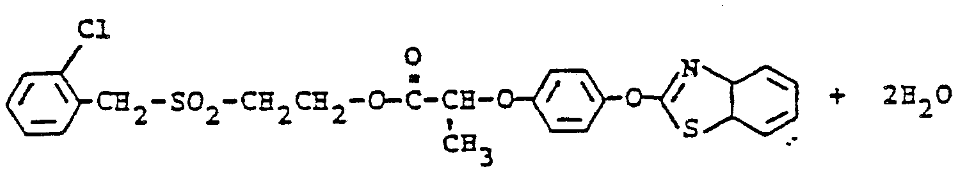

Die bei der Durchführung des erfindungsgemäßen Verfahrens (d) als Ausgangsstoffe benötigten Verbindungen der Formel Ia sind erfindungsgemäße Substanzen.

Bei der Durchführung des erfindungsgemäßen Verfahrens (d) können mit Ausnahme von Alkoholen vorzugsweise alle diejenigen Solventien und Verdünnungsmittel verwendet werden, die bereits im Zusammenhang mit dem erfindungsgemäßen Verfahren (a) genannt wurden.

Die Umsetzung nach dem erfindungsgemäßen Verfahren (d) kann innerhalb eines bestimmten Temperaturbereiches durchgeführt werden. Im allgemeinen arbeitet man bei Temperaturen zwischen — 20 °C und + 100 °C, vorzugsweise zwischen 0 °C und + 50 °C.

Die Umsetzung nach dem erfindungsgemäßen Verfahren (d) wird vorzugsweise unter atmosphärischem Druck durchgeführt ; sie kann jedoch auch unter erhöhtem oder vermindertem Druck vorgenommen werden.

Die erfindungsgemäßen Verbindungen können mit Vorteil zur Unkrautbekämpfung eingesetzt

werden, weil sie gegen Kulturpflanzen niedrige Toxizität und gute Selektivität besitzen, d. h. mit anderen Worten bei den üblichen Dosierungen keine Phytotoxizität gegenüber Kulturpflanzen ausüben. Die Herbizide gemäß der vorliegenden Erfindung zeigen eine hervorragende selektive Bekämpfungswirksamkeit insbesondere beim Einsatz gegen grasartige Unkräuter als Mittel zur Bodenbehandlung vor dem Auflaufen oder als Mittel zur Laub- und Bodenbehandlung.

Die erfindungsgemäßen Verbindungen der Formel I besitzen hohe Sicherheit und zeigen eine herausragende herbizide Wirksamkeit.

Das herbizide Spektrum dieser Verbindungen zeigt, daß sie eine starke herbizide Wirksamkeit entfalten gegen

Echinochloa crus-galli P. Beauv. var. oryzicola Ohwi,
Echinochloa crus-galli P. Beauv. var. caudata Kitagawa,
Echinochloa crus-galli P. Beauv. var. formosensis Ohwi, Sacciolepis indica Chase var. oryzetorum Ohwi,
Glyceria acutiflora torr.,
Glyceria natans Komorov.,
Polypogon fugax Steud.,
Panicum bisulcatum Thunb. sowie
Paspalum distichum L. und eine Hemmwirkung auf das Weiterwachsen perennierender Unkräuter ausüben.

Neben ihrer Wirkung gegen die oben beispielshaft angeführten Unkräuter zeigen die erfindungsgemäßen Verbindungen eine ausgezeichnete herbizide Wirkung gegenüber auf höher gelegenen Anbauflächen wachsenden grasartigen Unkräutern. Zu Beispielen für derartige Ackerunkräuter zählen

Echinochloa crus-galli P. Beauv.,
Agropyron tsukushiense Ohwi var. transiens Ohwi,
Poa annua L.,
Alopecurus aequalis Sobol. var. amurensis Ohwi,
Eleusine indica Gaertn.,
Digitaria adscendens Henr.,
Cynodon dactylon Pers.,
Setaria viridis P. Beauv.,
Setaria glauca P. Beauv. und
Avena fatua L.

Die erfindungsgemäßen Wirkstoffe können in vielen Kulturen sicher und ohne Schadwirkung als selektive Herbizide eingesetzt werden. — Als Beispiel seien die folgenden Kulturen genannt: Bohnen, Baumwolle, Möhren, Kartoffeln, Rüben, Kohl, Senf, Erdnüsse, Rettich, Tabak, Tomaten und Gurken.

Besonders vorteilhaft ist, daß die erfindungsgemäßen Wirkstoffe eine ausgeprägte und genaue Selektivität zwischen in Wasser wachsendem Reis und grasartigen Unkräutern aufweisen.

Für den Einsatz als Herbizide können die erfindungsgemäßen Wirkstoffe als solche unmittelbar nach dem Verdünnen mit Wasser oder in Form verschiedenartiger Präparate, wie sie gewöhnlich nach den bei der Herstellung von Agrochemikalien üblichen Verfahren unter Verwendung von landwirtschaftlich unbedenklichen Hilfsstoffen erhalten werden, angewandt werden. Beim praktischen Einsatz werden diese verschiedenartigen Präparate entweder unmittelbar oder nach dem Verdünnen mit Wasser in den gewünschten Konzentrationen ausgebracht.

Beispiele für die agrochemisch unbedenklichen Hilfsstoffe, auf die hier Bezug genommen wird, umfassen beispielsweise Verdünnungsmittel (Lösungsmittel, Streckmittel, Träger), oberflächenaktive Stoffe (Lösungsvermittler, Emulgatoren, Dispergiermittel, Netzmittel), Stabilisatoren, Haftmittel, Aerosol-Treibmittel und Synergisten.

Beispiele für die Lösungsmittel sind Wasser und organische Lösungsmittel, beispielsweise Kohlenwasserstoffe [z. B. n-Hexan, Petrolether, Naphtha, Erdöl-Fraktionen (z. B. Paraffinwachse, Kerosin, Leichtöle, Mittelöle, Schweröle), Benzol, Toluol und Xylole], halogenierte Kohlenwasserstoffe (z. B. Methylenchlorid, Tetrachlorkohlenstoff, Ethylenchlorid, Ethylendibromid, Chlorbenzol und Chloroform), Alkohole (z. B. Methylalkohol, Ethylalkohol, Propylalkohol und Ethylenglykol), Ether (z. B. Diethylether, Ethylenoxid und Dioxan), Alkoholether (z. B. Ethylenglycol-monomethylether), Ketone (z. B. Aceton und Isophoron), Ester (z. B. Ethylacetat und Amylacetat), Amide (z. B. Dimethylformamid und Dimethylacetamid) und Sulfoxide (z. B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z. B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylsulfonsäureester (z. B. Natriumlaurylsulfat), Arylsulfonsäure-Salze (z. B. Alkylarylsulfonsäure-Salze und Natriumalkylnaphthalinsulfonate), Bernsteinsäure-Salze und Salze von Schwefelsäureestern von Polyethylenglycolalkylarylethern, kationische oberflächenaktive Mittel wie Alkylamine (z. B. Laurylamin,

Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z. B. Polyoxyethylenalkylarylether und deren Kondensationsprodukte), Polyoxyethylenglycolester (z. B. Polyoxyethylenfettsäureester) und Ester mehrwertiger Alkohole (z. B. Polyoxyethylensorbitan-monolaurat) sowie amphotere oberflächenaktive Mittel.

Beispiele für andere Hilfsstoffe umfassen Stabilisatoren, Haftmittel (z. B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z. B. Trichlorofluoromethan, Dichlorofluoromethan, 1,2,2-Trichloro-1,1,2-trifluoroethan, Chlorbenzol, verflüssigtes Erdgas (LNG) sowie niedere Ether), die Verbrennung steuernde Mittel für Räuchermittel (z. B. Nitrite, Zink-Pulver und Dicyandiamid), Sauerstoff abgebende Mittel (z. B. Chlorate), wirkungsverlängernde Mittel, Dispersions-Stabilisatoren (z. B. Casein, Tragant, Carboxymethylcellulose (CMC) und Polyvinylalkohol (PVA) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öl-Präparate, benetzbare Pulver, lösliche Pulver, Suspensionen, Stäubemittel, Granulate und pulvrige Präparate.

Die Wirkstoffmenge kann in den anwendungsfertigen Formulierungen innerhalb eines größeren Bereiches variiert werden. Im allgemeinen liegt die Wirkstoffkonzentration zwischen 0,001 und 100 Gewichtsprozent, vorzugsweise zwischen 0,005 und 95 Gewichtsprozent.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,01 bis etwa 95 Gew.-%, vorzugsweise etwa 0,05 bis etwa 60 Gew.-%.

Der Gehalt des Wirkstoffes kann in geeigneter Weise je nach der Art des Präparats, dem Verfahren, Zweck, der Zeit und dem Ort seiner Anwendung, dem Zustand des Auftretens der Unkräuter etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, Fungiziden, Mitiziden, Nematiziden, Anti-Virus-Mitteln, anderen Herbiziden, Pflanzen-Wachstumsregulatoren und Lockstoffen [z. B. Organophosphat-Verbindungen, Carbamat-Verbindungen, Dithio (oder thiol) carbamat-Verbindungen, organischen Chlor-Verbindungen, Dinitro-Verbindungen, organischen Schwefel- oder metallorganischen Verbindungen, Antibiotika, substituierten Diphenylether-Verbindungen, Harnstoff-Verbindungen und Triazin-Verbindungen] und/oder Düngemitteln.

Die erfindungsgemäße Wirkstoffe enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.) und Bodenbehandlung (Vermischen mit dem Boden, Streuen etc.). Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmenge an Wirkstoff pro Flächeneinheit beträgt beispielsweise etwa 0,1 bis etwa 5 kg/ha, vorzugsweise etwa 0,2 bis etwa 2 kg/ha. In besonders gelagerten Fällen können oder sollten sogar die Aufwandmengen außerhalb des angegebenen Bereichs liegen.

Gemäß der vorliegenden Erfindung kann ein herbizides Mittel zur Verfügung gestellt werden, das als Wirkstoff eine Verbindung der allgemeinen Formel I sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung betrifft weiterhin ein Verfahren zur Unkrautbekämpfung, das darin besteht, daß eine Verbindung der allgemeinen Formel I allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf Unkräuter und/oder ihren Lebensraum aufgebracht wird.

Die vorliegende Erfindung wird durch die folgenden Beispiele näher erläutert, ist jedoch nicht auf diese speziellen Beispiele allein beschränkt.

## Herstellungsbeispiele

### Beispiel 1

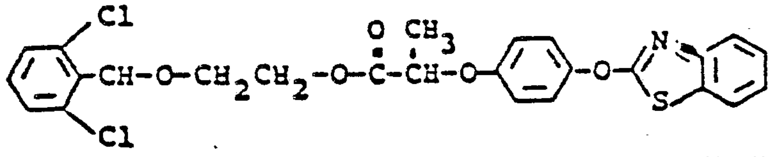

(Verbindung Nr. 1)

38,5 g 2(2,6-Dichlorobenzyloxy)ethyl-2-(4-hydroxyphenoxy)-propionat wurden in 200 ml Dimethylformamid gelöst, und 15,2 g Kaliumcarbonat wurden der Lösung zugesetzt. Die Mischung wurde 1 h bei 80 °C gerührt. Eine Lösung von 18,6 g 2-Chlorbenzothiazol in 50 ml Dimethylformamid wurde tropfenweise bei dieser Temperatur zu der entstandenen Mischung hinzugefügt. Nach der Zugabe wurde die Mischung bei der gleichen Temperatur zur Vervollständigung der Reaktion 2 h gerührt. Die Reaktionsmischung wurde auf Eiswasser gegossen, und das abgetrennte ölige Produkt wurde mit Toluol extrahiert. Die Toluol-Schicht wurde nacheinander mit einer 1-proz. wäßrigen Natriumhydroxid-Lösung und mit Wasser gewaschen, und dann wurde das Toluol unter vermindertem Druck abgedampft, wonach 2-(2,6-Dichlorobenzyloxy)ethyl-2-[4-benzothiazol-2-yloxy)phenoxy]propionat (43,5 g) als viskoses öliges Produkt erhalten wurde. $n_D^{25} = 1,6125$.

In Analogie zu der im Beispiel 1 angegebenen Methode wurden auch die in der folgenden Tabelle 1 aufgeführten Stoffe hergestellt.

Tabelle 1

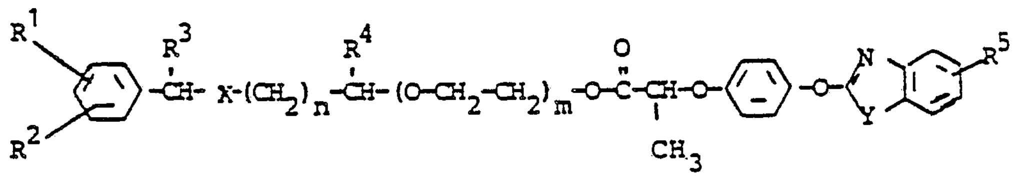

| Verbindung Nr. | $R^1$ | $R^2$ | $R^3$ | X | n | $R^4$ | m | Y | $R^5$ | Physikal. Konstante $n_D^{25}$ |
|---|---|---|---|---|---|---|---|---|---|---|
| 2 | 2-Cl | H | H | O | 1 | H | 0 | O | H | 1,5670 |
| 3 | 4-Cl | H | H | O | 1 | H | 0 | O | H | 1,5665 |
| 4 | 2-Cl | 6-Cl | H | O | 1 | H | 0 | O | H | 1,5915 |
| 5 | 2-F | H | H | O | 1 | H | 0 | O | H | 1,5460 |
| 6 | $2-CH_3$ | H | H | O | 1 | H | 0 | O | H | 1,5535 |
| 7 | $3-NO_2$ | H | H | O | 1 | H | 0 | O | H | 1,5750 |
| 8 | H | H | H | $-N-CH(CH_3)_2$ | 1 | H | 0 | O | H | 1,5560 |
| 9 | H | H | H | O | 1 | H | 0 | S | H | 1,5863 |
| 10 | H | H | H | O | 1 | H | 0 | S | 6-Cl | 1,5903 |
| 11 | 2-F | H | H | O | 1 | H | 0 | S | H | 1,5840 |
| 12 | 2-Cl | 6-Cl | H | O | 1 | H | 0 | S | H | 1,6135 |
| 13 | H | H | $-CH_3$ | O | 1 | H | 0 | S | H | 1,586 |
| 14 | H | H | H | O | 1 | H | 1 | S | H | 1,5750 |
| 15 | H | H | H | O | 1 | $-CH_3$ | 0 | S | H | 1,5872 |
| 16 | H | H | H | O | 2 | H | 0 | S | H | 1,5820 |
| 17 | H | H | H | S | 1 | H | 0 | S | H | 1,6130 |
| 18 | 2-Cl | H | H | S | 1 | H | 0 | S | H | 1,6192 |
| 19 | 2-Cl | H | H | $-SO_2-$ | 1 | H | 0 | S | H | 1,6300 |
| 20 | H | H | H | $-N-CH_3$ | 1 | H | 1 | S | H | 1,5910 |
| 21 | H | H | H | $-N-CH(CH_3)_2$ | 1 | H | 1 | S | H | 1,5918 |

Tabelle 1 (Fortsetzung)

| Verbindung Nr. | $R^1$ | $R^2$ | $R^3$ | X | n | $R^4$ | m | Y | $R^5$ | Physikal. Konstante $n_D^{25}$ *) |
|---|---|---|---|---|---|---|---|---|---|---|
| 22 | 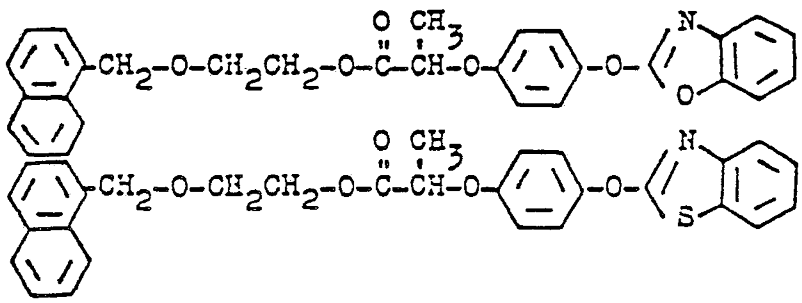 | | | | | | | | | 1,5995 |
| 23 | | | | | | | | | | 1,6226 |

*) Die für die Verbindung Nr. 6 angegebene physikalische Konstante ist $n_D^{20}$.

## Formulierungsbeispiele

### Beispiel 2

Benetzbares Pulver

15 Teile der erfindungsgemäßen Verbindung Nr. 6, 80 Teile eines Gemisches (1 : 5) aus pulveriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter und/oder ihren Lebensraum aufgesprüht.

### Beispiel 3

Emulgierbares Konzentrat

30 Teile der erfindungsgemäßen Verbindung Nr. 22, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter und/oder ihren Lebensraum aufgesprüht.

### Beispiel 4

Stäubemittel

2 Teile der erfindungsgemäßen Verbindung Nr. 5 und 98 Teile Tonpulver wurden pulverisiert und gemischt, wodurch ein Stäubemittel hergestellt wird. Dieses wird über Unkräutern und/oder ihrem Lebensraum ausgestreut.

### Beispiel 5

Granulat

25 Teile Wasser werden zu einer Mischung aus 10 Teilen der erfindungsgemäßen Verbindung Nr. 8, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40 °C bis 50 °C getrocknet, wodurch ein Granulat gebilldet wird. Dieses wird über Unkräutern und/oder ihrem Lebensraum ausgestreut.

Beispiel 6

Granulat

Ein Drehmischer wird mit 95 Teilen Tonmineral-Teilchen mit einer Teilchengrößen-Verteilung im Bereich von 0,2 bis 2 mm beschickt, und unter Drehen des Mischers werden 5 Teile der öligen erfindungsgemäßen Verbindung Nr. 15 zur gleichmäßigen Benetzung auf die Teilchen aufgesprüht, wodurch ein Granulat gebildet wird. Dieses wird über Unkräutern und/oder ihrem Lebensraum ausgestreut.

In den folgenden Verwendungsbeispielen wurde die nachnachstehend angegebene Verbindung als Vergleichssubstanz eingesetzt :

(A-1) 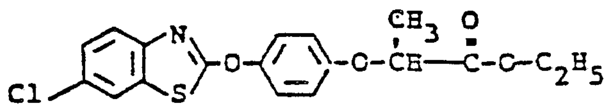

Beispiel 7 (Biologischer Test)

Prüfung der Wirkung gegen im Wasser wachsende Reisfeld-Unkräuter durch Behandlung des Laubs und des Bodens unter Bewässerungs-Bedingungen (Topf-Test) :

Wirkstoff-Präparat :

Träger : 5 Gewichtsteile Aceton ;
Emulgator : 1 Gewichtsteil Benzyloxypolyglycolether.

Zur Herstellung eines Wirkstoff-Präparats werden 2 Gew.-Teil jeder der aktiven Verbindungen mit dem Träger und Emulgator in den oben bezeichneten Mengen vermischt, und eine vorher festgelegte Menge des resultirenden emulgierbaren Konzentrats wurde mit Wasser verdünnt.

Test-Verfahren :

Reis Kulturboden wurde in Wagner-Töpfe (2 $dm^2$) gefüllt, und zwei Reis-Setzlinge (Varietät : Kinnampu) im 2- bis 3-Blattstadium (Pflanzenhöhe etwa 10 cm) wurden in jeden Topf umgepflanzt. Samen von Echinochloa crus-galli P. Beauv. var. oryzicola Ohwi, Echinochloa crus-galli P. Beauv. var. caudata Kitagawa, Sacciolepis indica Chase var. oryzetorum Ohwi und Panicum bisulcatum Thunb. wurden eingesät. Der Boden in den Töpfen wurde in feuchtem Zustand gehalten. Nachdem Echinochloa crus-galli P. Beauv. var. oryzicola Ohwi etwa bis zum 2-Blatt-Stadium gewachsen war (etwa 7 bis 9 Tage nach der Einsaat) wurden die Töpfe mit Wasser bis zu einer Tiefe von etwa 6 cm gefüllt. Eine vorher festgelegte Menge des Wirkstoffs wurde in Form einer Emulsion mittels einer Pipette zur Behandlung jedes Topfes zugegeben.

Nach der Behandlung wurden die Töpfe im Laufe von zwei Tagen mit einer Geschwindigkeit von 2 bis 3 cm pro Tag bewässert, während das Wasser aus den Töpfen heraussickern konnte. Danach wurde die Wassertiefe in den Töpfen bei etwa 3 cm gehalten. In der vierten Woche nach der Behandlung mit der Chemikalie wurden die herbizide Wirkung und der Grad der Phytotoxizität mit Hilfe einer Skala von 0 bis 5 nach dem folgenden Maßstab bewertet.

Bewertung der herbiziden Wirkung (bezogen auf die unbehandelte Fläche)

| | |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

Bewertung der Phytotoxizität gegenüber Reispflanzen im Wasser (bezogen auf die unbehandelte Fläche)

| | |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Ergebnisse sind in Tabelle 2 aufgeführt.

Tabelle 2

| Verbindung Nr. | Wirkstoff-Menge kg/ha | Herbizide Wirkung A | B | C | D | Phytotoxizität gegen Reis |
|---|---|---|---|---|---|---|
| 1 | 1,0 | 5 | 5 | 5 | 5 | 0 |
| 5 | 1,0 | 5 | 5 | 5 | 5 | 0 |
| 6 | 1,0 | 5 | 5 | 5 | 5 | 0 |
| 11 | 1,0 | 5 | 5 | 5 | 5 | 0 |
| 12 | 1,0 | 5 | 5 | 5 | 5 | 0 |
| 15 | 0,5 | 5 | 5 | 5 | 5 | 0 |
| 16 | 0,5 | 5 | 5 | 5 | 5 | 0 |
| 18 | 1,0 | 5 | 5 | 5 | 5 | 0 |
| 21 | 1,0 | 5 | 5 | 5 | 5 | 0 |
| Vergleich: | | | | | | |
| A-1 | 1,0 | 5 | 5 | 5 | 5 | 4 |
| | 0,5 | 5 | 5 | 5 | 5 | 3 |
| | 0,1 | 3 | 3 | 4 | 4 | 2 |

Anmerkungen

1. Die Symbole A bis D bezeichnen die nachstehenden Unkräuter:
   A: Echinochloa crus-galli P. Beauv. var. oryzicola Ohwi,
   B: Echinochloa crus-galli P. Beauv. var. caudata Kitagawa,
   C: Sacciolepis indica Chase var. oryzetorum Ohwi und
   D: Panicum bisulcatum Thunb.
2. Die Nummern der Verbindungen entsprechen den hierin im Vorstehenden angegebenen.

Es wurde sichergestellt, daß andere aktive Verbindungen der allgemeinen Formel I gemäß der vorliegenden Erfindung als die oben beispielhaft aufgeführten eine selektive Bekämpfungswirkung gegen grasartige Unkräuter besitzen, ohne Phytotoxizität gegen Reis zu zeigen.

**Patentansprüche**

1. Substituierte Phenoxyalkancarbonsäureester der Formel I,

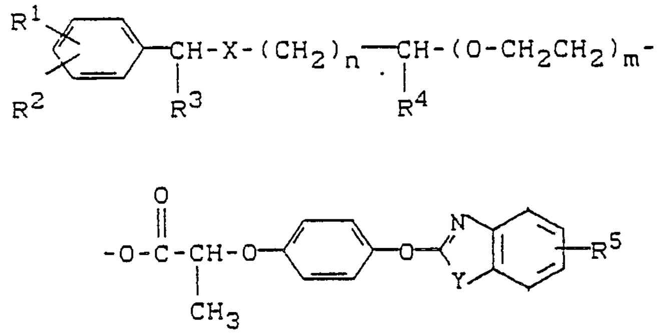

(I)

in welcher

$R^1$ und $R^2$ unabhängig voneinander für Wasserstoff, Fluor, Chlor, Brom, Jod, Alkyl mit 1 bis 6 Kohlenstoffatomen oder Nitro stehen, oder

$R^1$ und $R^2$ gemeinsam mit dem Benzolring, and den sie gebunden sind, einen Naphthalin-Ring bilden,

$R^3$ und $R^4$ unabhängig voneinander für Wasserstoff oder Methyl stehen,

m für 0 oder 1 steht,

n für 1 oder 2 steht,

X für Sauerstoff, Schwefel, eine Sulfonylgruppe oder eine Gruppe der Formel

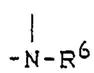

steht, worin $R^6$ für Alkyl mit 1 bis 6 Kohlenstoffatomen steht,

$R^5$ für Wasserstoff, Fluor, Chlor, Brom oder Jod steht und

Y für Sauerstoff oder Schwefel steht.

2. 2-(2-Chlorobenzyloxy)ethyl-2-[4-(benzothiazol-2-yl-oxy)phenoxy]propionat der Formel

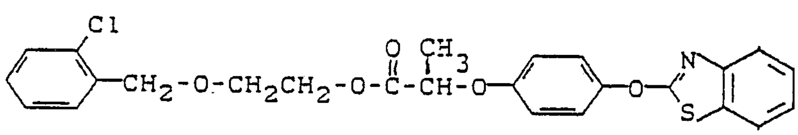

3. Verfahren zur Herstellung substituierter Phenoxyalkancarbonsäureester der Formel I,

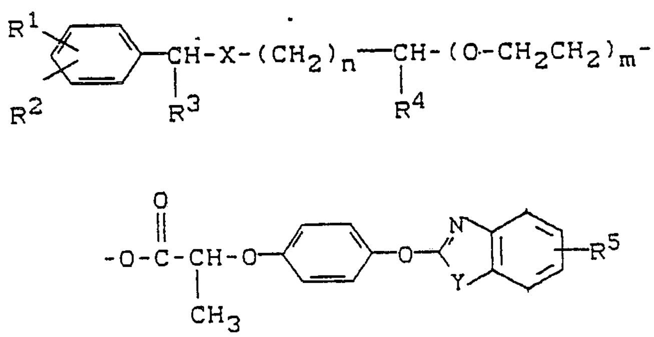

(I)

in welcher

$R^1$ und $R^2$ unabhängig voneinander für Wasserstoff, Fluor, Chlor, Brom, Jod, Alkyl mit 1 bis 6 Kohlenstoffatomen oder Nitro stehen, oder

$R^1$ und $R^2$ gemeinsam mit dem Benzolring, an den sie gebunden sind, einen Naphthalin-Ring bilden,

$R^3$ und $R^4$ unabhängig voneinander für Wassertoff oder Methyl stehen,

m für 0 oder 1 steht,

n für 1 oder 2 steht,

X für Sauerstoff, Schwefel, eine Sulfonylgruppe oder eine Gruppe der Formel

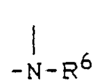

steht, worin $R^6$ für Alkyl mit 1 bis 6 Kohlenstoffatomen steht,
$R^5$ für Wasserstoff, Fluor, Chlor, Brom oder Jod steht und
Y für Sauerstoff oder Schwefel steht,
dadurch gekennzeichnet, daß man
a) Verbindungen der Formel II,

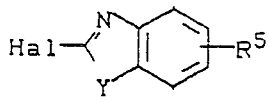
(II)

in welcher
$R^5$ und Y die oben angegebene Bedeutung haben und
Hal für Halogen steht,
mit Verbindungen der Formel III,

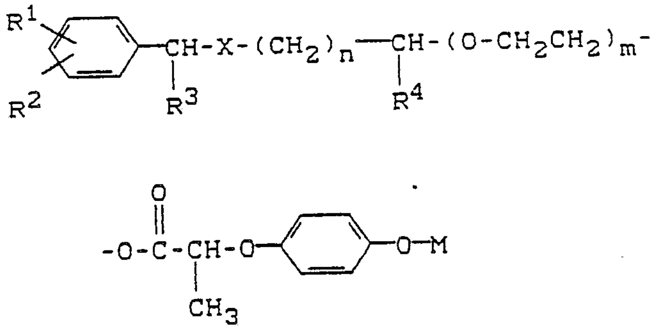
(III)

in welcher
$R^1$, $R^2$, $R^3$, $R^4$, X, m und n die oben angegebene Bedeutung haben und
M für Wasserstoff oder ein Alkalimetallatom steht,
umsetzt,
oder
b) Verbindungen der Formel IV,

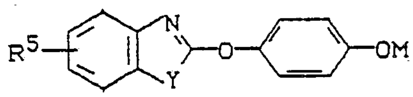
(IV)

in welcher
$R^5$ und Y die oben angegebene Bedeutung haben und
M für Wasserstoff oder ein Alkalimetallatom steht,
mit Verbindungen der Formel V,

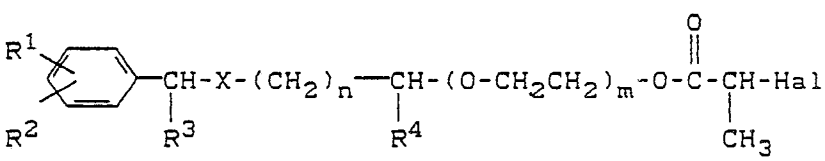
(V)

in welcher
$R^1$, $R^2$, $R^3$, $R^4$, X, m und n die oben angegebene Bedeutung haben und
Hal für Halogen steht,
umsetzt,
oder
c) Verbindungen der Formel VI,

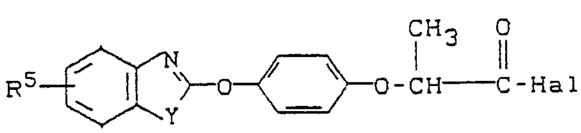
(VI)

in welcher

$R^5$ und Y die oben angegebene Bedeutung haben und
Hal für Halogen steht,
mit Verbindungen der Formel VII,

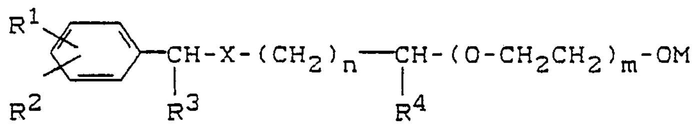

(VII)

in welcher
$R^1$, $R^2$, $R^3$, $R^4$, X, m und n die oben angegebene Bedeutung haben und
M für Wasserstoff oder ein Alkalimetallatom steht,
umsetzt,
oder
d) Verbindungen der Formel Ia,

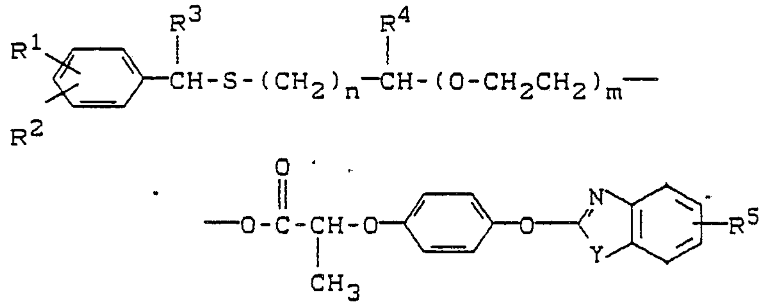

(Ia)

in welcher $R^1$, $R^2$, $R^3$, $R^4$, $R^5$, Y, m und n die oben angegebene Bedeutung haben, mit Wasserstoffperoxid umsetzt.

4. Herbizide Mittel, gekennzeichnet durch einen Gehalt an mindestens einem substituierten Phenoxyalkancarbonsäureester der Formel I gemäß den Ansprüchen 1 bis 3.

5. Verwendung von substituierten Phenoxyalkancarbonsäureestern der Formel I gemäß den Ansprüchen 1 bis 3 zur Bekämpfung von Unkräutern.

6. Verfahren zur Bekämpfung von Unkräutern, dadurch gekennzeichnet, daß man substituierte Phenoxyalkancarbonsäureester der Formel I gemäß den Ansprüchen 1 bis 3 auf die Unkräuter und/oder ihren Lebensraum ausbringt.

7. Verfahren zur Herstellung herbizider Mittel, dadurch gekennzeichnet, daß man substituierte Phenoxyalkancarbonsäureester der Formel I gemäß den Ansprüchen 1 bis 3 mit Streckmitteln und/oder oberflächenaktiven Stoffen vermischt.

**Claims**

1. Substituted phenoxyalkanecarboxylic acid esters of the formula I

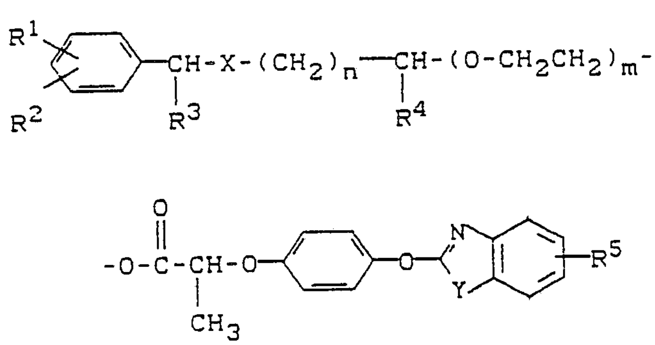

(I)

in which
$R^1$ und $R^2$ independently of one another represent hydrogen, fluorine, chlorine, bromine, iodine, alkyl with 1 to 6 carbon atoms or nitro, or
$R^1$ and $R^2$, together with the benzene ring to which they are bonded, form a naphthalene ring,
$R^3$ und $R^4$ independently of one another represent hydrogen or methyl,

m represents 0 or 1,
n represents 1 or 2,
X represents oxygen, sulphur, a sulphonyl group or a group of the formula

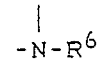

wherein $R^6$ represents alkyl with 1 to 6 carbon atoms,
$R^5$ represents hydrogen, fluorine, chlorine, bromine or iodine and
Y represents oxygen or sulphur.

2. 2-(2-Chlorobenzyloxy)ethyl-2-[4-benzothiazol-2-yloxy)phenoxy]propionate of the formula

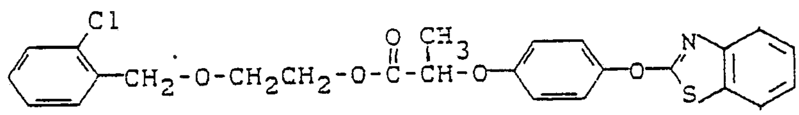

3. Process for the preparation of substituted phenoxyalkanecarboxylic acid esters of the formula I

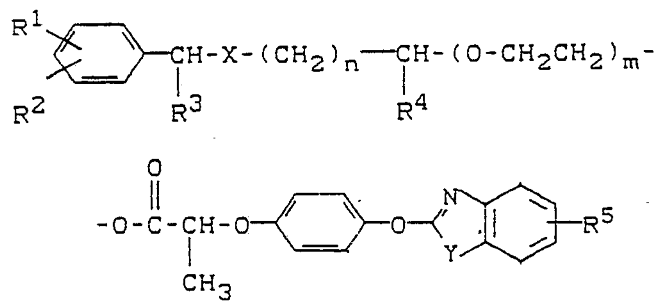

(I)

in which
$R^1$ and $R^2$ independently of one another represent hydrogen, fluorine, chlorine, bromine, iodine, alkyl with 1 to 6 carbon atoms or nitro, or
$R^1$ and $R^2$, together with the benzene ring to which they are bonded, form a naphthalene ring,
$R^3$ and $R^4$ independently of one another represent hydrogen or methyl,
m represents 0 or 1,
n represents 1 or 2,
X represents oxygen, sulphur, a sulphonyl group or a group of the formula

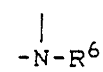

wherein $R^6$ represents alkyl with 1 to 6 carbon atoms,
$R^5$ represents hydrogen, fluorine, chlorine, bromine or iodine and
Y represents oxygen or sulphur,
characterized in that
a) compounds of the formula II

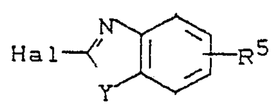

(II)

in which
$R^5$ and Y have the abovementioned meaning and
Hal represents halogen,
are reacted with compounds of the formula III

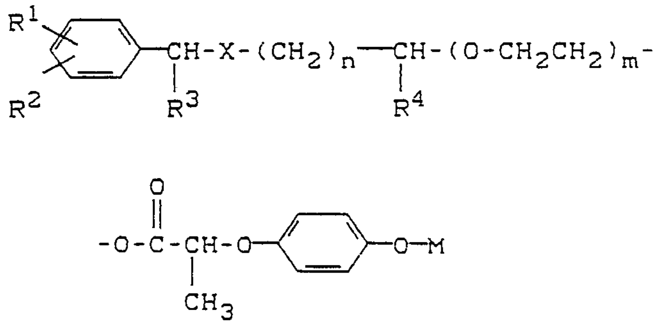

(III)

in which
$R^1$, $R^2$, $R^3$, $R^4$, X, m and n have the abovementioned meaning and
M represents hydrogen or an alkali metal atom,
or
b) compounds of the formula IV

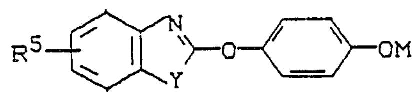

(IV)

in which
$R^5$ and Y have the abovementioned meaning and
M represents hydrogen or an alkali metal atom,
are reacted with compounds of the formula V

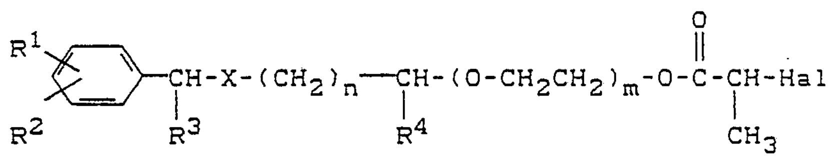

(V)

in which
$R^1$, $R^2$, $R^3$, $R^4$, X, m and n have the abovementioned meaning and
Hal represents halogen,
or
c) compounds of the formula VI

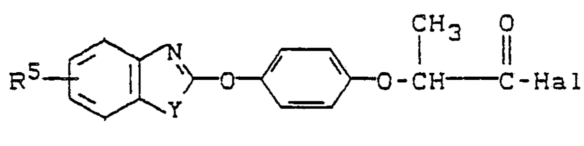

(VI)

in which
$R^5$ and Y have the abovementioned meaning and
Hal represents halogen,
are reacted with compounds of the formula VII

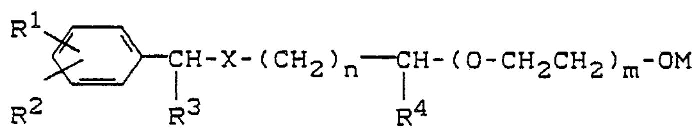

(VII)

in which
$R^1$, $R^2$, $R^3$, $R^4$, X, m and n have the abovementioned meaning and
M represents hydrogen or an alkali metal atom,
or
d) compounds of the formula Ia

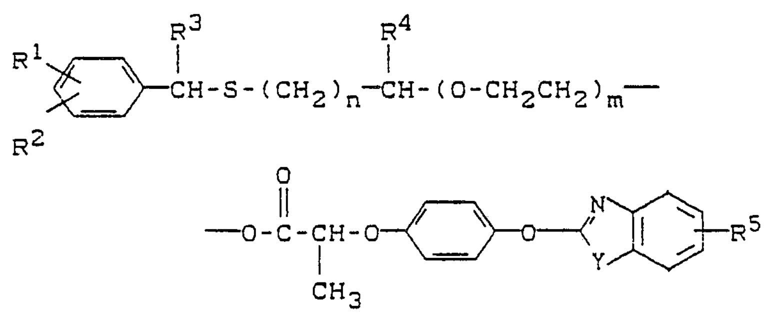

(Ia)

in which $R^1$, $R^2$, $R^3$, $R^4$, $R^5$, Y, m and n have the abovementioned meaning,
are reacted with hydrogen peroxide.

4. Herbicidal agents, characterized in that they contain at least one substituted phenoxyalkanecarboxylic acid ester of the formula I according to Claims 1 to 3.

5. Use of substituted phenoxyalkanecarboxylic acid esters of the formula I according to Claims 1 to 3 for combating weeds.

6. Method of combating weeds, characterized in that substituted phenoxyalkanecarboxylic acid esters of the formula I according to Claims 1 to 3 are applied to the weeds and/or their environment.

7. Process for the preparation of herbicidal agents, characterized in that substituted phenoxyalkanecarboxylic acid esters of the formula I according to Claims 1 to 3 are mixed with extenders and/or surface-active substances.

**Revendications**

1. Esters d'acides phénoxyalcanecarboxyliques substitués de formule I

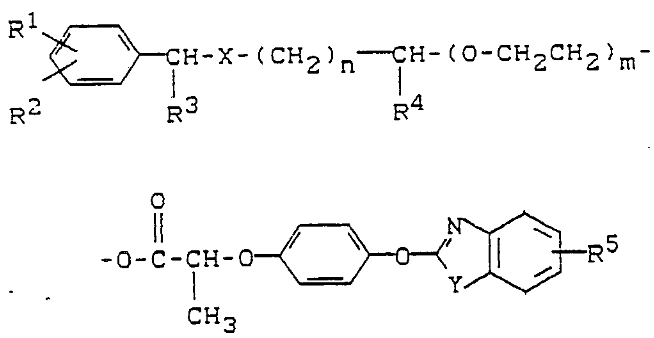

(I)

dans laquelle

$R^1$ et $R^2$ représentent, indépendamment l'un de l'autre, l'hydrogène, le fluor, le chore, le brome, l'iode, un groupe alkyle ayant 1 à 6 atomes de carbone ou un groupe nitro,

$R^1$ et $R^2$ forment un noyau de naphtalène conjointement avec le noyau benzénique auquel ils sont liés,

$R^3$ et $R^4$ représentent, indépendamment l'un de l'autre, l'hydrogène ou le groupe méthyle,

m a la valeur 0 ou 1,

n a la valeur 1 ou 2,

X est l'oxygène, le soufre, un groupe sulfonyle ou un groupe de formule

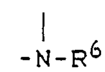

dans laquelle $R^6$ est un groupe alkyle ayant 1 à 6 atomes de carbone,

$R^5$ représente l'hydrogène, le fluor, le chlore, le brome ou l'iode et

Y est l'oxygène ou le soufre.

2. Le 2-[4-(benzothiazole-2-yloxy)phénoxy]-propionate de 2-(2-chlorobenzyloxy)éthyle de formule

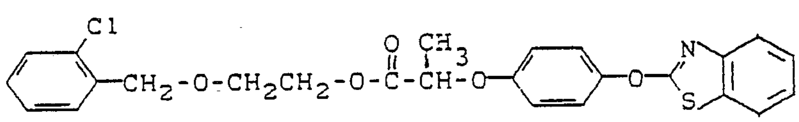

3. Procédé de préparation d'esters d'acides phénoxyalcanecarboxyliques substitués de formule I

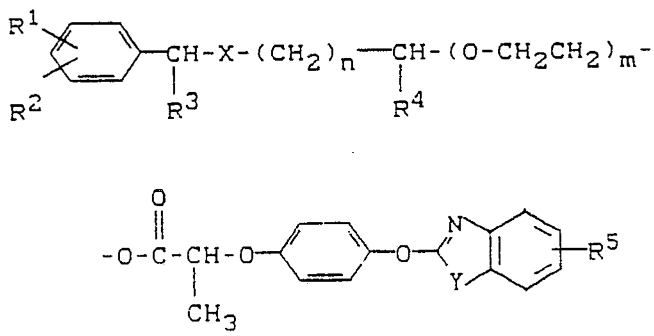

(I)

dans laquelle

$R^1$ et $R^2$ représentent, indépendamment l'un de l'autre, l'hydrogène, le fluor, le chlore, le brome, l'iode, un groupe alkyle ayant 1 à 6 atomes de carbone ou un groupe nitro,

$R^1$ et $R^2$ forment un noyau de naphtalène conjointement avec le noyau benzénique auquel ils sont liés,

$R^3$ et $R^4$ représentent, indépendamment l'un de l'autre, l'hydrogène ou le groupe méthyle

m a la valeur 0 ou 1,

n a la valeur 1 ou 2,

X est l'oxygène, le soufre, un groupe sulfonyle ou un groupe de formule

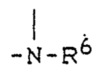

dans laquelle $R^6$ est un groupe alkyle ayant 1 à 6 atomes de carbone,

$R^5$ représente l'hydrogène, le fluor, le chlore, le brome ou l'iode et

Y est l'oxygène ou le soufre,

caractérisé en ce qu'on fait réagir

a) des composés de formule II

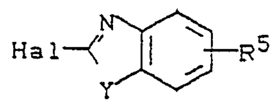 (II)

dans laquelle

$R^5$ et Y ont la définition indiquée ci-dessus et

Hal représente un halogène,

avec des composés de formule III

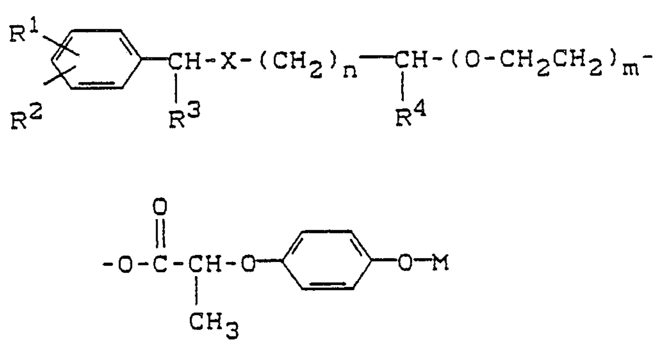 (III)

dans laquelle

$R^1$, $R^2$, $R^3$, $R^4$, X, m et n ont la définition indiquée ci-dessus et

M représente l'hydrogène ou un atome de métal alcalin,

ou bien

b) des composés de formule IV

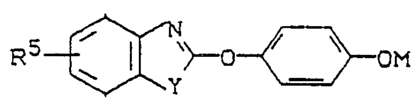 (IV)

dans laquelle

$R^5$ et Y ont la définition indiquée ci-dessus et

M représente l'hydrogène ou un atome de métal alcalin,

avec des composés de formule V

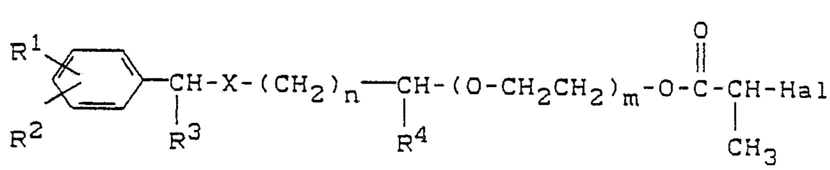 (V)

dans laquelle
$R^1$, $R^2$, $R^3$, $R^4$, X, m et n ont la définition indiquée ci-dessus et
Hal représente un halogène,
ou bien
c) des composés de formule VI

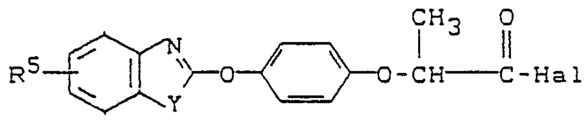

(VI)

dans laquelle
$R^5$ et Y ont la définition indiquée ci-dessus et
Hal représente un halogène,
avec des composés de formule VII

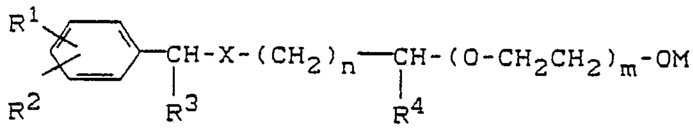

(VII)

dans laquelle
$R^1$, $R^2$, $R^3$, $R^4$, X, m et n ont la définition indiquée ci-dessus et
M représente l'hydrogène ou un atome de métal alcalin,
ou bien
d) des composés de formule Ia

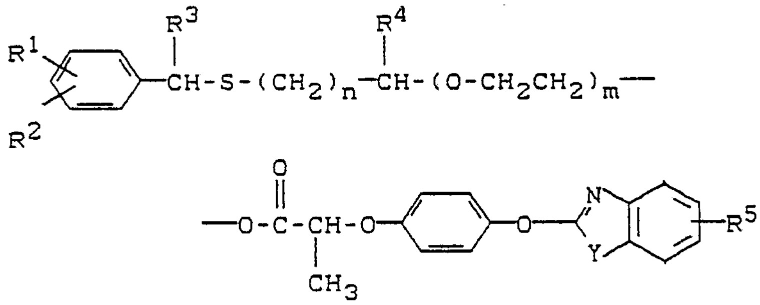

(Ia)

dans laquelle $R^1$, $R^2$, $R^3$, $R^4$, $R^5$, Y, m et n ont la définition indiquée ci-dessus, avec le peroxyde d'hydrogène.

4. Compositions herbicides, caractérisées par une teneur en au moins un ester d'acide phénoxyalcanecarboxylique substitué de formule I suivant les reventications 1 à 3.

5. Utilisation d'esters d'acides phénoxyalcanecarboxyliques substitués de formule I suivant les revendications 1 à 3 pour combattre des mauvaises herbes.

6. Procédé pour combattre des mauvaises herbes, caractérisé en ce qu'on applique des esters d'acides phénoxyalcanecarboxyliques substitués de formule I suivant les revendications 1 à 3 sur les mauvaises herbes et/ou sur leur milieu.

7. Procédé de préparation de compositions herbicides, caractérisé en ce qu'on mélange des esters d'acides phénoxyalcanecarboxyliques substitués de formule I suivant les revendications 1 à 3 avec des diluants et/ou des agents tensio-actifs.